Europäisches Patentamt

European Patent Office

Office européen des brevets

(19)

(11) **EP 1 284 143 A1**

(12) **EUROPEAN PATENT APPLICATION**

(43) Date of publication:
**19.02.2003 Bulletin 2003/08**

(51) Int Cl.⁷: **A61K 38/40**, A61K 9/08

(21) Application number: **01830545.8**

(22) Date of filing: **17.08.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | • **Paladino, Grazia**<br>**95020 Lavinaio-Aci S.Antonio (Catania) (IT)**<br>• **Marino, Clara**<br>**95020 Lavinaio-Aci S.Antonio (Catania) (IT)**<br>• **Blanco, Anna Rita**<br>**95020 Lavinaio-Aci S.Antonio (Catania) (IT)** |
| (71) Applicant: **Sifi S.p.A**<br>**95020 Lavinaio-Aci S.Antonio (IT)** | • **Amico, Carla**<br>**95020 Lavinaio-Aci S.Antonio (Catania) (IT)**<br>• **Enea, Vincenzo**<br>**95020 Lavinaio-Aci S.Antonio (Catania) (IT)** |
| (72) Inventors:<br>• **Mazzone, Maria Grazia**<br>**95020 Lavinaio-Aci S.Antonio (Catania) (IT)**<br>• **Marrano, Manuela**<br>**95020 Lavinaio-Aci S.Antonio (Catania) (IT)**<br>• **Asero, Antonino**<br>**95020 Lavinaio-Aci S.Antonio (Catania) (IT)** | (74) Representative: **Long, Giorgio et al**<br>**Jacobacci & Partners S.p.A.**<br>**Via Senato, 8**<br>**20121 Milano (IT)** |

(54) **A process for the preparation of pharmaceutical formulations containing lactoferrin description**

(57)    The present invention relates to a process for the preparation of pharmaceutical formulations containing the glycoprotein lactoferrin and/or its peptide fragments in therapeutical quantities, in which said process is performed at a temperature between 0 and 25°C in order to keep the bacterial load at less than 10 CFU per 100 ml, comprising the following steps:

a) preparation of an aqueous solution for pharmaceutical formulation with a pH between 5 and 8;
b) addition of the glycoprotein lactoferrin to the solution obtained after completion of step a), while stirring at no more than 250 rpm;

c) filtering of the solution obtained after the completion of step b) thorough filters with pores ranging from 0.1 to 0.45 μm.

Said process allows to obtain pharmaceutical formulations suitable for carrying the glycoprotein lactoferrin or its fragments in a stable manner for a medium-long period of time to guarantee its long and safe storage.

**EP 1 284 143 A1**

Printed by Jouve, 75001 PARIS (FR)

**Description**

The present invention relates to a process for the preparation of pharmaceutical formulations containing the glycoprotein lactoferrin and/or its peptide fragments.

In particular, the present invention relates to a process for the preparation of pharmaceutical formulations containing the glycoprotein lactoferrin and/or its peptide fragments suitable for pharmaceutical use, in particular in the ophthalmic sector in formulations such as eye drops, gels and other release systems for said lactoferrin.

Advances in the knowledge of the structure of endogenous proteins and peptides and their role in numerous physiological processes, as well as the use of biotechnological techniques to synthesize them have led to the development of peptides and proteins as therapeutics.

However, problems of stability, chemical and physical, in solutions and lack of the desirable characteristics for adequate tissue distribution and absorption have limited the clinical applications of peptides and proteins for topical use. In order to make these proteins available as drugs it is essential to formulate them in a stable, safe and effective way. Therefore, one of the challenges is to be able to formulate pharmaceutical preparations that can preserve the structural integrity of the protein, both conformational and chemical, during the period of industrial preparation and of use of the final product.

Lactoferrin, a glycoprotein of the transferrin family with a molecular weight of about 80 kDA, may be obtained from natural sources through isolation and purification (e.g. milk of mammals including human, colostrum, saliva, gastroenteric and pancreatic secretions, tears) [Peter Ferenc Levay, Margaretha Viljoen Lactoferrin: A general Review *Haematologica* (1995) 80: 252-267, B. Lönnerdal and S. Iyer Lactoferrin:

Molecular Structure and Biological Function *Annu. Rev. Nutr.* (1995) 15: 93-110], through genetic engineering techniques (e.g. recombinant expression of the protein), through direct production in genetically modified animals, or through chemical synthesis.

In particular, lactoferrin is found in high concentrations in human tears relative to other body fluids (except mammalian milk) and its concentration ranges from 1 to 2 g/l, thus representing about 25% of all lacrimal proteins.

It is known that the concentration of lactoferrin in tears tends to diminish with age and also in certain ocular pathologies such as Sjögren's Syndrome and keratoconjunctivitis sicca (KCS), to the point that the determination of the level of lactoferrin is used as one of the clinical parameters in the diagnosis of KCS [Mark Ballow, P.C. Donshik Pamela Rapacz and Lisa Samartino Tear Lactoferrin Levels in Patients with External Inflammatory Ocular Disease *Invest. Ophthalm. Vis. Sci* . (1987), 28(3): 543-545, C.J. McCollum et al. Rapid Assay of Lactoferrin in Keratoconjunctivitis Sicca. *Cornea* (1994) 13(6): 505-508, S. DA Dalt, A. Moncada, R. Priori, G. Valesini, P. Pivetti-Pezzi The Lactoferrin Tear Test in the Diagnosis of Sjogren's Syndrome. *European J. of Ophthalmol.* (1996) 6(3): 284-286].

Numerous works in the international scientific literature describe the various properties of lactoferrin such as its antimicrobial, bacteriostatic and bactericidal activity [Roland R. Arnold, Michael F. Cole, and Jerry R. McGhee A Bactericidal Effect for Human Lactoferrin *Science* (1977) 197: 263-265; Roland R. Arnold, Michael Brewer and Joseph J. Gauthier Bactericidal Activity of Human Lactoferrin: Sensitity of a Variety of Microorganisms *Infection and Immunity* (1980) 28(3): 893-898; Richard T. Ellison III and Theodore J. Giehl Killing of Gram-negative Bacteria by Lactoferrin and Lysozyme. *J. Clin. Invest.*(1991) 88: 1080-1091; R. S. Bhimani, Y. Vendrov and P. Furmanski Influence of Lactoferrin Feeding and Injection against Systemic Staphylococcal Infections in Mice *J. of Appl. Microb.* (1999) 86: 135-144; E. C. Leitch and M.D.P. Willcox Lactoferrin Increase the Susceptibility of *S. epidermidis* Biofilms to Lysozyme and Vancomycin *Curr. Eye Res.* (1999) 19(1): 12-19], its immunomodulatory activity or its cytoprotective activity towards cells subjected to various types of oxidative stress involving the generation of free radicals through iron-catalyzed reactions [Ab Kuizenga, Nico J. Van Haeringen and Aize Kijlstra Inhibition of Hydroxyl Radical Formation by human Tears. *Invest. Ophthalm. Vis. Sci.* (1987), 28:305-314; S. Shimmura, M. Suematsu, M. Shimoyama, K. Tsubota, Y. Oguchi, Y. Ishimura Subthreshold UV radiation-induced peroxide formation in cultured corneal epithelial cells: the protective effects of lactoferrin. *Exp. Eye Res.* (1996) 63: 519- 526; S. Shimmura, M. Shimoyama, M. Hojo, K. Urayama and K. Tsubota. Reoxygenation Injury in a Cultured Corneal Epithelial cell Line Protected by the Uptake of Lactoferrin. *Invest. Ophthalm. Vis. Sci.* (1998), 39(8): 1346-1351].

Some published patents describe the use of lactoferrin as a drug for indications such as corneal wound healing (US Patent 5,561,109), treatment of bacterial infections (US Patent 5,834,424) viral infections (US 5,725,864) or tumors (EP090331).

In addition, mixtures intended to serve as lacrimal substitutes containing lactoferrin together with other proteins and/or other biologically active components present in tears have been described (US 4,911,933 - US 5,652,209 - PCT WO 99/06022).

In particular, international patent application PCT WO99/06065 describes an aqueous formulation containing lactoferrin and an inorganic or a polyvalent inorganic acid or a salt thereof. This formulation is stable for a short period of about twenty days, in addition analytic tests to demonstrate the biological stability of the formulation are not reported.

It is evident that the formulation described in the above-mentioned application for international patent is stable for a period too limited to assure an acceptable shelf-life for the product.

The problem to be addressed by the present invention is therefore to provide a process for the preparation of pharmaceutical formulations suitable for the vehiculation of the glycoprotein lactoferrin and its peptide fragments such as are stable for a medium-long period in order to guarantee a prolonged and safe storage.

Said problem is solved with a process for the preparation of pharmaceutical formulations containing the glycoprotein lactoferrin and/or its peptide fragments as described in the main annexed claim.

In particular, through numerous and accurate experimentations, it was surprisingly found that, by operating under certain conditions it is possible to obtain a pharmaceutical formulation containing lactoferrin such that it remains stable and active for a period of at least 24 months.

The process for the preparation of pharmaceutical formulations containing the glycoprotein lactoferrin and/or its peptide fragments was realized at a temperature between 0 and 25° C.

The aforesaid process includes the following steps:

  a) preparation of an aqueous solution for pharmaceutical formulations with a pH between 5 and 8.
  b) addition of the glycoprotein lactoferrin to the solution obtained after the step a) while stirring at no more than 250 rpm.
  c) filtering under pressure of the solution obtained after the step b) through filters with pores ranging from 0.1 to 0.45 μm.

The value assigned to the stirring speed was determined using a pilot plant. Nonetheless, the suitable modifications of said value in production plants are standard practice for technicians in this sector.

It should be borne in mind, during the preparation process, that the formulation should contain a bacterial load of less than 10 CFUs (colony forming units) per 100 ml.

The above-mentioned process should be carried out preferably at a temperature between 2 and 15° C, more preferably between 4 and 12° C.

The aqueous solution has a preferred pH between 6.6 and 7.8.

The process may include the addition of a buffer made of an acid-base pair. Preferably, such buffer is chosen from the group consisting of: phosphate, phosphate citrate, phosphate-bicarbonate, Tris HCL.

A quantity between 0.5 and 150 mM of buffer, preferably between 2 and 105 mM, is added.

It should be borne in mind that the addition of any substance should be carried out only after the substance added previously is totally dissolved.

Such process may also require the addition of one or more non-ionic and/or ionic isotonizing agent. In particular, the ionic isotonizing agent is preferably chosen within the group consisting of sodium, potassium, magnesium, and calcium chloride or from a mixture of them. Instead the non-ionic isotonizing agent of choice is preferably glycerol or mannitol.

The isotonizing agents are added in quantities between 15 mM and 230 mM, preferably between 70 mM and 205 mM (expressed as moles of NaCl).

In addition, during the process, various substances may be added such as viscosity-enhancers, gelling agents, absorption promoters, and/or stabilizing agents.

In particular, viscosing or gelling agents with a molecular weight between 100,000 and 5,000,000 Dalton are preferably chosen among sodium hyaluronate, xanthan gum, Kollidon®, Lutrol F127®, Carbopol®, cellulose and cellulose derivates. Moreover, if the mentioned viscosing and gelling agents are filterable, then they can be added before filtration; otherwise, it is carried out after filtration and, in this case, said agents should be sterile.

Among the absorption enhancing agents, chitosan, azone, cetrimide, EDTA, sodium lauryl sulphate, polysorbates, mono and diglycerides, are preferred.

The stabilizing agents are preferably chosen from the group consisting of polyethylene glycols, polypropylene glycols, ascorbates, ionic and non-ionic tensioactives, citrates, sulphites, retinol, β-carotene, and tocopherol.

According to the present invention, the process for the preparation of the multidose pharmaceutical formulation may require the addition of an adequate quantity of preservative such as for instance, benzalkonium chloride, cetrimide, parabens, polyexamethyleneguanide, chlorhexidine, chlorobutanol, sorbates and/or other excipients commonly used in pharmaceutical formulations. Such preservatives are added in quantities between 0.1% and 0.0001%, preferably between 0.01% and 0.002%.

The filtering step is preferably carried out using filters with pores between 0.1 and 0.2μm, under pressure with air, or preferably with an inert gas.

Lactoferrin may be added, in particular, in quantities between 0.002 and 4% by weight in grams per 100ml of the total volume of the final formulation. Preferably, lactoferrin is added in quantities between 0.05 and 3%, more preferably between 0.1 and 2% by weight in grams per 100ml of the total volume of the final formulation. In the following example (1), in particular, a stable pharmaceutical formulation of lactoferrin in saline solution was prepared.

**Example 1**

A volume of water injection-grade equivalent to 80% of the total volume is introduced into a graduated container. Afterwards, the organic salts, the citrate and the glycerol when present, are solubilized in this order, according to the quantities reported in example 2-7. Special care is taken not to add the next component until the previous one is totally dissolved. If included in the formulation, the polymer, or the viscosing or gelling agent is added, and left to swell for the time necessary for total solubilization. Keeping the temperature under control, preferably between 4 and 12° C, the lactoferrin is added, and the solution is stirred at no more than 250 rpm until complete dissolution is achieved. In the case of a multidose preparation, the antimicrobial preservative is added. Subsequently, sterile filtration under nitrogen through filters with low protein retention, and with pores between 0.1 and 0.2 $\mu$m is performed. Finally, the solution is distributed in plastic or glass containers.

A further object of the present invention is to provide a pharmaceutical formulation comprising the glycoprotein lactoferrin and/or its peptide fragments in therapeutical quantities.

Such formulation may be obtained following the above described processes.

In particular, the formulation is characterized by an osmolarity between 150mOsmol/kg and 320mOsmol/kg, preferably between 190 mOsmol/kg and 307 mOsmol/kg.

Possible buffers, isotonizing, viscosing or gelling agents, absorption enhancing agents, stabilizing agents and preservatives correspond to those previously described in the process for the preparation of said pharmaceutical formulation.

The quantities of such substances, including lactoferrin, also correspond to those previously described.

Lactoferrin and its peptide fragments may be obtained through isolation and purification of natural sources such as, mammalian milk, by means of genetic engineering techniques such as the recombinant expression of the protein, through direct production in genetically modified animals or through chemical synthesis.

According to the invention, the pharmaceutical formulations containing lactoferrin and/or its peptide fragments were prepared as aqueous and/or gel ophthalmic formulations.

Some specific instantiations of these formulations are reported hereunder as non-limitative examples of this invention.

The units of measurement regarding the quantity of substance employed are expressed as weight in grams of each substance per 100 ml of water per pharmaceutical solution and in the corresponding mM.

Three preferred different formulations have been prepared for each embodiment in view of the presence or not of some substances.

## Example 2

- Lactoferrin (extractive or recombinant origin) 0.2 % (w/v);

- TPS*

| * Phosphate buffer | | | Form.1 | Form.2 | Form.3 |
|---|---|---|---|---|---|
| | Composition % | mM | | | |
| $Na_2HPO_4$ $12H_2O$ | 1.9100 | 53.34 | X | X | X |
| $NaH_2PO_4$ $H_2O$ | 0.2100 | 15.22 | X | X | X |
| NaCl | 0.4400 | 75.29 | X | X | X |
| Sodium hyaluronate MW=2.5±0.5MD | 0.15 | | - | X | X |
| Benzalkonium Chloride | 0.005 | | - | - | X |
| $H_2O$ | q.s. to 100ml | | X | X | X |

X= component present

| pH=7.69 |
|---|
| mOsm/kg=292 |

## Example 3

- Lactoferrin (extractive or recombinant origin) 0.2 % (w/v);

|  | Composition % | mM | Form.1 | Form.2 | Form.3 |
|---|---|---|---|---|---|
| Tris.HCl | 0.063 | 4 | X | X | X |
| NaCl | 0.87 | 150 | X | X | X |
| Sodium hyaluronate MW=2.5±0.5MD | 0.15 | | - | X | X |
| Benzalkonium chloride | 0.005 | | - | - | X |
| $H_2O$ | q.s. to 100ml | | X | X | X |

| pH=7.45 |
|---|
| mOsm/kg=297 |

## Example 4

- Lactoferrin (extractive or recombinant origin) 0.2 % (p/v)

**- Citrate buffer\*\***

| **\*\*Citrate buffer** | Composition % | mM | Form.1 | Form.2 | Form.3 |
|---|---|---|---|---|---|
| $Na_3C_6H_5O_7\,2H_2O$ | 3.0234 | 103 | X | X | X |
| $C_6H_8O_7\,H_2O$ | 0.0255 | 1.2 | X | X | X |
| Sodium hyaluronate MW=2.5±0.5MD | 0.15 | | - | X | X |
| Benzalkonium chloride | 0.005 | | - | - | X |

| H$_2$O | q.s. to 100ml | | X | X | X |
|---|---|---|---|---|---|

| pH=7.15 |
|---|
| mOsm/Kg=289 |

## Example 5

- Lactoferrin (extractive or recombinant origin) 0.2 % (w/v)

- **TPS/mannitol***

| ***phosphate/mannitol buffer | | | Form. 1 | Form. 2 | Form. 3 |
|---|---|---|---|---|---|
| | **Composition %** | mM | | | |
| Na$_2$HPO$_4$ 12H$_2$O | 1.9070 | 53.25 | X | X | X |
| NaH$_2$PO$_4$ H$_2$O | 0.2102 | 15.23 | X | X | X |
| Mannitol | 2.3674 | 130 | X | X | X |
| Sodium hyaluronate MW=2.5±0.5MD | 0.15 | | — | X | X |
| Benzalkonium chloride | 0.005 | | — | — | X |
| H$_2$O | q.s. to 100ml | | X | X | X |

| pH=7.47 |
|---|
| mOsm/Kg=289 |

## Example 6

- Lactoferrin (extractive or recombinant origin) 0.2 % (w/v)

- **BSS/glycerol****

| ****BSS/glycerol 1% | | | Form. 1 | Form. 2 | Form. 3 |
|---|---|---|---|---|---|
| Balanced Saline Solution | **Composition %** | mM | | | |

| | | | | | |
|---|---|---|---|---|---|
| Na$_2$HPO$_4$ 12H$_2$O | 0.0890 | 2.5 | X | X | X |
| NaH$_2$PO$_4$ H$_2$O | 0.0069 | 0.5 | X | X | X |
| NaCl | 0.3508 | 60 | X | X | X |
| KCl | 0.1500 | 20 | X | X | X |
| MgCl$_2$ 6H$_2$O | 0.0134 | 0.6 | X | X | X |
| CaCl$_2$ 2H$_2$O | 0.0085 | 0.6 | X | X | X |
| Na$_3$C$_6$H$_5$O$_7$ 2H$_2$O | 0.0590 | 2 | X | X | X |
| Glycerol | 1 | 109 | X | X | X |
| Sodium hyaluronate MW=2.5±0.5MD | 0.15 | | - | X | X |
| Benzalkonium chloride | | | - | - | X |
| H$_2$O | q.s. to 100ml | | X | X | X |

| |
|---|
| pH=7.48 |
| mOsm/Kg=278 |

## Example 7

- Lactoferrin (extractive or recombinant origin) 0,2 % (w/v)

- - **BSS/NaCl*****

| *****BSS/NaCl | | | Form. 1 | Form. 2 | Form. 3 |
|---|---|---|---|---|---|
| Balanced Saline Solution | **Composition %** | mM | | | |
| Na$_2$HPO$_4$ 12H$_2$O | 0.0890 | 2.5 | X | X | X |
| NaH$_2$PO$_4$ H$_2$O | 0.0069 | 0.5 | X | X | X |
| NaCl | 0.6670 | 114 | X | X | X |
| KCl | 0.2100 | 28 | X | X | X |
| MgCl$_2$ 6H$_2$O | 0.0134 | 0.6 | X | X | X |
| CaCl$_2$ 2H$_2$O | 0.0085 | 0.6 | X | X | X |

| $Na_3C_6H_5O_7\ 2H_2O$ | 0.0590 | 2 | X | X | X |
|---|---|---|---|---|---|
| Sodium hyaluronate MW=2.5±0.5MD | 0.15 | | - | X | X |
| Benzalkonium chloride | | | - | - | X |
| $H_2O$ | q.s. to 100ml | | X | X | X |

| pH=7.40 |
|---|
| mOsm/Kg=278 |

For comparisons, formulations corresponding to those of examples 2-7 were prepared adding thimerosal and/ or in pH conditions higher than 8. The results show that lactoferrin is not stable for a period longer than three months.

The next examples were carried out to evaluate the stability, tolerability and efficacy of pharmaceutical formulations comprising lactoferrin.

The safety of the formulations was tested both *in vitro*, on SIRC cell cultures, and *in vivo* on rabbits (see examples 9 and 10). The various tests carried out on cell cultures, made possible to surprisingly detect a modulatory activity of lactoferrin on the proliferation of the cells, depending on which phase of the growth curve the cell culture resides. The phenomenon is particularly evident in cells exposed to a medium less rich in nutrients.

The iron uptake test of lactoferrin has proven to be a good analytical test to monitor the biological activity of the protein during the stability period of the formulated solutions. The test is able to detect differences in the protein's capability to sequester iron.

An additional correlation between the stability of lactoferrin and its biological activity is obtained with a microbiological test able to detect possible differences in the capability of lactoferrin to inhibit the growth of a given bacterial strain (*Escherichia coli* ATCC 25922).

**Example 8**

Stability at various temperatures (4°C, 25°C, 40°C)

The analytical methods used for the characterization of the stability of lactoferrin in ophthalmic formulations were calorimetry, gel electrophoresis (SDS PAGE) (qualitative analysis), size exclusion chromatography (qualitative analysis), ionic exchange chromatography (quantitative analysis) (IE assay), inverse phase chromatography (quantitative analysis) (RP assay), iron uptake biological test, quantitative titer in terms of the capability of the protein to saturate in the presence of $Fe^{3+}$ ($\Delta Up_{Fe3+}$ which is defined as the difference between the saturability and the saturation percentage of the protein).

$$\Delta Up_{Fe3+} = \%\ \text{saturability} - \%\ \text{saturation}$$

Where

%saturab.= $Abs_{465nm}$ after interaction with $Fe^{3+}$ / Conc.Lf %

%saturat.= $Abs_{465nm}$ before interaction with $Fe^{3+}$ / Conc.Lf %

according to Stowell et al. *Biochem. J.* (1991) 276; 349-355.

An additional analytical method used as quantitative assay is the capacity of Lf to inhibit the bacterial growth of *E. coli* ATCC 25922 (see example 9).

The formulations indicated, stored at temperatures of 4°C and 25°C ± 2°C under relative humidity condition of 75% ± 5%, are stable and active even after 24 months. As a non-limiting example, the stability of the formulation with sodium hyaluronate with a molecular weight of 2.5±0.5 MD without antimicrobic preservative, reported in example 5, is shown in the following table (table 1). After confirming the consistency of the data obtained with the various analytical methods for the protein under formulation the following were chosen as routine techniques SDS PAGE, RP

assay, $\Delta Up_{Fe3+}$, microbiological test, viscosity and assay of the polymer if present as viscosing or gelling agent.

## TABLE 1

| Test | | 0 | 3 | 6 | 9 | 12 | 18 | 24 |
|---|---|---|---|---|---|---|---|---|
| Test | | Months | | | | | | |
| Appearance | | Complies[a] | Complies[a] | Complies[a] | Complies[a] | Complies[a] | Complies[a] | Complies[a] |
| pH | | 7.52 | 7.42 | 7.43 | 7.48 | 7.50 | 7.48 | 7.37 |
| mOsm/Kg | | 285 | 286 | 287 | 300 | 297 | 292 | 291 |
| Molecular Weight NaHA (MD) | | - | 2.453 | 2.185 | 2.193 | 2.131 | 2.104 | 1.917 |
| NaHA Assay (% decl.) | | - | 98.5 | - | 96.3 | 98.7 | 99.2 | 101.3 |
| Viscosity (cP) (Sh rate sec$^{-1}$ 178.2) [b] | | 25.6 | - | 23.4 | 23.1 | - | - | 21.6 |
| Lactoferrin Analysis | RP Assay (% decl.) | 97.00 | 94.20 | 98.38 | 97.94 | 99.21 | 98.77 | 99.88 |
| Lactoferrin Analysis | SDS PAGE | Complies[c] | - | Complies[c] | - | Complies[c] | Complies[c] | Complies[c] |
| Lactoferrin Analysis | %$\Delta Up_{Fe3+}$ | 46.57 | 43.62 | 43.86 | 43.85 | 43.65 | 40.68 | 40.00 |
| Microbiological Test | Optical Density | 0.79 | - | - | - | 0.81 | 0.80 | 0.80 |
| Sterility | | Sterile | - | - | - | - | - | Sterile |

- Complies[a]: Clean and viscous solution, from colorless to light pink.

- Complies[c]: Gel electrophoresis shows a main band with a molecular weight around 81000D that identifies lactoferrin in the active dimer conformation, and 2 minor bands at 52000D and 48000D detectable by overloading the gel, that constitute impurities.

EP 1 284 143 A1

**Example 9**

Stability microbiological test

The test evaluates the antibacterial activity of the lactoferrin contained in the formulations subjected to stability tests relative to the formulation freshly prepared.

The experiment was carried out by plating *E. coli* ATCC 25922 on TSA plates (Tryptic Soy Agar) and incubating at 35°C. The colonies were collected after about 20 hours and were suspended in a 10ml of physiological solution until a suspension with an optical density (OD) equal to one ($\lambda$=660nm) was obtained.

The culture medium (Bacto-Casitone Difco base medium) is prepared at 2%, in such way that, mixed at a 1:1 ratio with the tested solution, it has a final concentration equal to 1%.

A freshly prepared solution containing lactoferrin at the same initial concentration as that of the solutions placed under stability, the vehicle used to formulate the lactoferrin, and solutions at different stability time points containing the protein were used for the test. Each of the solutions to be tested was mixed with an equal volume of the base medium, in a 1:1 ratio.

The test is carried out in microplates of 96 microwells, each containing 198$\mu$l of either experimental sample or control, to which 2$\mu$l of bacterial suspension standardized at a concentration with OD=1 is added.

The microplate is then incubated at 35°C for 24 hours.

OD is read with an spectrophotometer fitted with a microplate adaptor.

The inhibition percentage of bacterial growth by lactoferrin is calculated with the following formula:

$$\text{Growth inhibition \%}=100- (A/Bx100)$$

Where:

A= OD (optical density) difference of the samples at 24 hours relative to T0;
B= OD (optical density) difference of the control at 24 hours relative to T0.

Results:

As can be clearly seen in Fig.1 the solutions under stability maintain the same capacity to inhibit the growth of E. coli ATCC 25922 as that of the freshly prepared solutions.

**Comparison at different times between fresh made
solutions of lactoferrin and solutions kept in stability**

Fig. 1

## Example 10

*In vitro* safety-efficacy on SIRC cells

The following experiment was carried out to verify the tolerability of different concentrations of lactoferrin in a cellular system.

The concentration curve used is: 0, 5, 50, 100 $\mu$M. The formulation containing lactoferrin was solubilized in culture medium and kept in contact with the cells during the whole experiment.

Two culture media were used: 1) DMEM a medium rich in nutrients and 2) BME, a basic medium. The latter in particular lacks iron salts.

Method:

1. Plates of 12 microwells with 30000 cells/per well
2. Treatment with Lf (lactoferrin); controls: cells treated with DMEM or BME in absence of Lf
3. Cell count using Trypan blue coloration, at T1, T3 and T6 days after the first treatment.

Results:

$T_1$: no difference between the control cells and the lactoferrin treated cells at any of the tested concentrations.

$T_3$: control cells treated with DMEM are star shaped and with reduced dimensions, control cells treated with BME are star shaped and with more markedly reduced dimensions that the cells treated with DMEM; cells treated with 5, 50 e 100 $\mu$M of lactoferrin both in DMEM and BME keep normal morphology. Cells treated with 100 $\mu$M of lactoferrin are numerically inferior, but this result is not statistically significant.

$T_6$: in the DMEM group the cells treated with 100 $\mu$M of lactoferrin are reduced relative to the control, while no difference is observed in the BME groups treated with 100 $\mu$M of lactoferrin. In both DMEM and BME groups a proliferative activity up to a concentration of 50 $\mu$M is observed.

The cells in the BME group treated with 50 $\mu$M of lactoferrin were numerically superior, with a statistically significant difference ($p<0.05$).

The cells treated with 5 μM, 10 μM and 50 μM are present in clones with the typical epithelioid morphology, a well-dispersed chromatin with a 1/3 nucleus/cytoplasm ratio.

In conclusion, the different concentrations of lactoferrin are well tolerated in the *in vitro* model used. The decrease in cells numbers observed in the DMEM group is not statistically significant; furthermore, it is not observed in the cells cultivated with BME. On the contrary the cells cultivated with BME, poorer in nutrients, show a proliferative activity that is not present in the group treated with the same concentration of lactoferrin but using a medium richer in nutrients.

In the following tables, the toxicity of lactoferrin on cells grown on two different culture media is evaluated.

## TABLE 2

### Toxicity curve of lactoferrin in SIRC cells grown in DMEM

The values represent the number of cells per microwell

|  | Control | 5μM | 50μM | 100μM |
|---|---|---|---|---|
| T0 | 9275±1170 | -- | -- | -- |
| T1 | 7300±3500 | 3777±631 | 12200±1385 | 9722±2839 |

| T3 | 23125±5543.35 | 21666.6±3547.29 | 18333.3±2516.6 | 14666.6±4310.8 |
|---|---|---|---|---|
| T6 | 29500±9389.7 | 60166.6±17250.6* | 38833.3±19237.5 | 14000±9124.1 |

* statistically significant data relative to the control

## TABLE 3

### Toxicity curve of lactoferrin in SIRC cells grown in BME

The values represent the number of cells per microwell

|  | Control | 5μM | 50μM | 100μM |
|---|---|---|---|---|
| T0 | 9250±500 | -- | -- | -- |
| T1 | 12166±1154 | 10333±2843 | 12000±3774 | 12333±1443 |
| T3 | 27000±4509.2 | 23333.3±4252.4 | 23722±3860 | 21666.6±8751.2 |
| T6 | 71250±11644 | 100333.3±21807.9 | 127833.3±38001 * | 70333.3±24901.5 |

* statistically significant data relative to the control

$p < 0.05$ t-test

**Example 11**

Ocular tolerability at 28 days

Eight New Zealand (male) rabbits, 1.8-2.3 kg, were randomly distributed in groups (n = 2) that were to be treated topically with 2% lactoferrin or with placebo according to the following scheme:

| Group | Treatment | |
|---|---|---|
| | **Right eye** | **Left eye** |
| 1 | Eye Drops | Not treated |
| 2 | Placebo | Not treated |

Eight treatments were performed the first day and four in the following days. One drop (50 µl) of 2% lactoferrin or placebo was administered in the conjunctival sac of each animal. After the administration, the eyelids were kept closed for several seconds to reduce the loss of solution and to enable a homogeneous distribution in the eye. The statistical difference between each treated group and the respective control was evaluated using the Mann-Whitney test.

Ocular clinical examination was evaluated with a slit lamp at 0, 1\4, 1\2, 1, 2, 3, 4, 5, 6, 7, 8, 24, 48, 72, 96, 168, 240, 360, 480, and 672 hours after the beginning of the study. Ocular tolerability/toxicity was defined using the score system developed by Draize: the clinical signs were registered in the conjunctiva (congestion, edema, exudates), cornea (opacity) and iris (dilatation). The severity of the observed signs was assigned according to a score from 0 to 3 (0=normal, 1=mild, 2=moderate, 3=severe).

After the clinical examination, the corneas were analyzed by SEM (Scanning Electron Microscopy)

Based on the clinical signs, no evidence of toxic effects was detected after the use of 2% lactoferrin during a 28-days treatment. The Draize test showed no significant differences between the 2% lactoferrin eye drops and the placebo. The cornea examination by SEM did not detect any particular modification of the cornea. In conclusion, the 2% lactoferrin eye drop formulation is well tolerated after ocular topical instillation during 28 days in the albino rabbits.

As described before, during the process for the preparation of the formulation described in the present invention it is important that the bacterial load be lower than 10 CFU per 100ml of formulation.

To this end, it is possible to work alternatively under sterile condition or in environmental conditions such that the mentioned load is not present and substantially with sterile substances. In this way, it is possible to work around or above room temperature and the final filtering can be performed with less selective filters or be omitted.

It should be noted, however, that good results were also obtained, independently of the bacterial load, with pharmaceutical formulations comprising the glycoprotein lactoferrin and/or its peptide fragments in therapeutical quantities, a buffer in quantities between 0.5 and 150 mM, preferably between 2mM and 105 mM, and an isotonizing agent or a mix of ionic and/or non-ionic isotonizing agents in quantities between 15 mM and 150 mM, so as to obtain a pH between 5 and 8.0, preferably between 6.6 and 7.8, and an osmolarity between 150 and 320 mOsmol/kg, preferably between 190 and 307 mOsmol/kg.

Other substances and quantities that could be used in the aforementioned formulation correspond to those previously described.

According to the invention the formulations can be prepared advantageously also for other pharmaceutical applications, for instance in dermatology.

More generally, the formulations containing lactoferrin described above can be profitably applied to other glycoproteins.

In this way, it becomes possible to obtain pharmaceutical formulations containing a variety of glycoproteins with excellent stability and storage characteristics.

**Claims**

1. Process for the preparation of pharmaceutical formulations containing the glycoprotein lactoferrin and/or its peptide fragments in therapeutical quantities, in which said process is performed at a temperature between 0 and 25°C in order to keep the bacterial load at less than 10 CFU per 100 ml, comprising the following steps:

   a) preparation of an aqueous solution for pharmaceutical formulation with a pH between 5 and 8;
   b) addition of the glycoprotein lactoferrin to the solution obtained after completion of step a), while stirring at no more than 250 rpm;
c) filtering of the solution obtained after the completion of step b) thorugh filters with pores ranging from 0.1 to 0.45 μm.

2. Process according to claim 1, in which said process is performed at a temperature between 2 and 15°C.

3. Process according to claim 1 or 2 in which said process is performed at a temperature between 4 and 12°C.

4. Process according to any of the claims from 1 to 3, in which the pH of the aqueous solution ranges between 6.6 and 7.8.

5. Process according to any of the claims from 1 to 4, in which the filtering step c) is performed under pressure with air or an inert gas and sterile filters with pores between 0.1 e 0.2 μm.

6. Process according to any of the claims from 1 to 5, comprising a step in which a buffer made of a acid base pair is added.

7. Process according to claim 6, in which said buffer is chosen within the group consisting of phosphate, phosphate citrate, phosphate-bicarbonate, Tris HCl.

8. Process according to claim 6 or 7, in which said buffer is added in quantities between 0.5 and 150 mM.

9. Process according to claims 6, 7 or 8, in which said buffer is added in quantities between 2 and 105 mM.

10. Process according to any of the claims from 1 to 9, comprising a step in which ionic and/or non-ionic isotonizing agents are added.

11. Process according to claim 10, in which said ionic isotonizing agents are chosen within the group made of sodium, potassium, magnesium, calcium chloride, while the non-ionic isotonizing agents are chosen between glycerol and mannitol.

12. Process according to claim 10 or 11, in which the isotonizing agents are added in quantities between 15 and 230 mM.

13. Process according to any of the claims from 10 to 12, in which the isotonizing agents are added in quantities between 70 and 205 mM.

14. Process according to any of the claims from 1 to 13, comprising a step in which viscosing or gelling agents with a molecular weight among 100,000 and 5,000,000 Daltons are added; said step taking place before filtering if said agents are filterable, or after filtrations if said agents are non-filterable, in which case said agents are sterile.

15. Process according to claim 14, in which said viscosing or gelling agents are chosen between sodium hyaluronate, xanthan gum, Kollidon, Lutrol F127, Carbopol, cellulose and cellulose derivates.

16. Process according to any of the claims from 1 to 15, comprising a step in which tissue absorption enhancing agents are added.

17. Process according to claim 16, in which said tissue absorption enhancing agents are chosen among chitosan, azone, cetrimide, EDTA, sodium laurylsulphate, polysorbates, mono and diglycerides.

18. Process according to any of the claims from 1 to 17, comprising a step in which stabilizing agents are added.

19. Process according to claim 18, in which said stabilizers are chosen among polyethylene glycols, polypropylene glycols, ascorbates, ionic and non-ionic tensioactives, citrates, sulphites, retinol, β-carotene, and tocopherol.

20. Process according to any of the claims from 1 to 19, comprising the step in which a preservative is added.

21. Process according to claim 20, in which said preservative is chosen among benzalkonium chloride, cetrimide, parabens, polyexamethyleneguanide, chlorhexidine, chlorobutanol, sorbates and/or other excipients commonly used in therapeutical formulations in general.

22. Process according to any of the claims from 1 to 21, in which lactoferrin is added in quantities between 0.002 e 4% (grams per 100 ml of total volume).

23. Process according to any of the claims from 1 to 23, in which lactoferrin is added in quantities between 0.05 e 3% grams per 100 ml of total volume of said pharmaceutical formulations.

24. Process according to any of the claims from 1 to 23, in which lactoferrin is added in quantities between 0.1 e 2% grams per 100 ml of total volume of said pharmaceutical formulations.

25. Pharmaceutical formulation comprising the glycoprotein lactoferrin and/or its peptide fragments obtainable according to the process of the claims from 1 to 24.

26. Pharmaceutical formulation according to claim 25, in which said formulation is such as to keep the homeostasis of the eye.

27. Process for the preparation of a pharmaceutical formulation including the glycoprotein lactoferrin comprising the following preparations carried out under sterile conditions:

a) preparation of a sterile aqueous solution for pharmaceutical formulations with a pH between 5.0 and 8, including a buffer made of an acid base pair and an isotonizer, or a mix of ionic and/or non-ionic isotonizers; b) addition, while stirring at no more than 250 rpm, of the glycoprotein lactoferrin to the solution obtained after step a).

28. Pharmaceutical formulation comprising the glycoprotein lactoferrin and/or its peptide fragments obtainable according to the process of claim 28.

29. Pharmaceutical formulation comprising the glycoprotein lactoferrin and/or its peptide fragments in quantities between 0.02 and 4% grams per 100 ml of total volume, a buffer in quantities between 0.5 and 150 mM and an isotonizer, or a mix of ionic and/or non-ionic isotonizers, in quantities between 15 mM and 230 mM.

30. Pharmaceutical formulation comprising:

- Lactofenin 0.2 % (w/v)
- **BSS/glycerol****

| ****BSS/glycerol 1% | | |
|---|---|---|
| Balanced Saline Solution | **Composition %** | mM |
| $Na_2HPO_4$ $12H_2O$ | 0.0890 | 2.5 |
| $NaH_2PO_4$ $H_2O$ | 0.0069 | 0.5 |
| NaCl | 0.3508 | 60 |
| KCl | 0.1500 | 20 |
| $MgCl_2$ $6H_2O$ | 0.0134 | 0.6 |
| $CaCl_2$ $2H_2O$ | 0.0085 | 0.6 |
| $Na_3C_6H_5O_7$ $2H_2O$ | 0.0590 | 2 |
| Glycerol | 1 | 109 |
| Sodium hyaluronate MW=2.5±0.5MD | 0.15 | |
| $H_2O$ | q.s. to 100ml | |

**31.** Pharmaceutical formulation comprising:

- Lactoferrin 0.2 % (w/v)
  **BSS/NaCl*****

| *****BSS/NaCl | | |
|---|---|---|
| Balanced Saline Solution | **Composition %** | mM |
| $Na_2HPO_4$ $12H_2O$ | 0.0890 | 2.5 |
| $NaH_2PO_4$ $H_2O$ | 0.0069 | 0.5 |
| NaCl | 0.6670 | 114 |
| KCl | 0.2100 | 28 |
| $MgCl_2$ $6H_2O$ | 0.0134 | 0.6 |
| $CaCl_2$ $2H_2O$ | 0.0085 | 0.6 |
| $Na_3C_6H_5O_7$ $2H_2O$ | 0.0590 | 2 |
| Sodium hyaluronate | 0.1500 | |
| MW=2.5±0.5MD | | |
| $H_2O$ | q.s. to 100ml | |

**European Patent Office**

# PARTIAL EUROPEAN SEARCH REPORT

**Application Number**

which under Rule 45 of the European Patent Convention EP 01 83 0545
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | EP 1 000 627 A (SANTEN PHARMA CO LTD) 17 May 2000 (2000-05-17) * page 3; example 1 * | 1-31 | A61K38/40 A61K9/08 |
| X | EP 1 075 837 A (S I F I SOCIETA IND FARMACEUTI) 14 February 2001 (2001-02-14) * page 4 - page 5; example 1 * | 1-31 | |
| A | EP 0 826 373 A (SANTEN PHARMA CO LTD) 4 March 1998 (1998-03-04) * column 4; table 1 * | 1-31 | |
| A | US 5 198 419 A (ANDO KUNIO ET AL) 30 March 1993 (1993-03-30) * column 4, line 27 - line 34 * | 1-31 | |

**TECHNICAL FIELDS SEARCHED (Int.Cl.7)**

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 14 February 2002 | Boulois, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**INCOMPLETE SEARCH
SHEET C**

Application Number

EP 01 83 0545

Claim(s) searched incompletely:
    1,25,28,29

Reason for the limitation of the search:

Present claims 1,25,28 and 29 refer to "peptide fragments" of
lactoferrin. These "peptide fragments" are not defined in the present
application, and therefore must be considered to be unclear and not
supported technically by the description ( Article 84 EPC ). The
consequence is that a lack of clarity within the meaning of Article 84
EPC arises to such an extent as to render a meaningful search of this
part of the claims impossible. Consequently, the search has been carried
out for those parts of the application which do appear to be clear,
namely 1-31 as far as they concerned lactoferrin. Peptide fragments of
lactoferrin were not searched.

EP 1 284 143 A1

**ANNEX TO THE EUROPEAN SEARCH REPORT
ON EUROPEAN PATENT APPLICATION NO.**

EP 01 83 0545

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2002

| Patent document cited in search report | | Publication date | Patent family member(s) | | Publication date |
|---|---|---|---|---|---|
| EP 1000627 | A | 17-05-2000 | JP | 11049698 A | 23-02-1999 |
| | | | EP | 1000627 A1 | 17-05-2000 |
| | | | WO | 9906065 A1 | 11-02-1999 |
| EP 1075837 | A | 14-02-2001 | IT | MI991803 A1 | 09-02-2001 |
| | | | EP | 1075837 A2 | 14-02-2001 |
| | | | JP | 2001089378 A | 03-04-2001 |
| | | | US | 6277829 B1 | 21-08-2001 |
| EP 0826373 | A | 04-03-1998 | JP | 3118584 B2 | 18-12-2000 |
| | | | JP | 8301785 A | 19-11-1996 |
| | | | EP | 0826373 A1 | 04-03-1998 |
| | | | WO | 9635448 A1 | 14-11-1996 |
| US 5198419 | A | 30-03-1993 | JP | 3181421 A | 07-08-1991 |
| | | | CA | 2031746 A1 | 09-06-1991 |
| | | | DE | 69004941 D1 | 13-01-1994 |
| | | | DE | 69004941 T2 | 07-07-1994 |
| | | | EP | 0431933 A1 | 12-06-1991 |